# EUROPEAN PATENT SPECIFICATION

(11) **EP 3 587 431 B1**
(45) Date of publication and mention of the grant of the patent: **23.12.2020**
(21) Application number: 17898032.2
(22) Date of filing: 08.03.2017
(51) Int. Cl.: C07D 493/08

(54) **METHOD FOR PREPARING LEVOGLUCOSENONE BY CATALYTIC PYROLYSIS OF BIOMASS**
VERFAHREN ZUR HERSTELLUNG VON LEVOGLUCOSENON DURCH KATALYTISCHE PYROLYSE VON BIOMASSE
PROCÉDÉ DE PRÉPARATION DE LÉVOGLUCOSÉNONE PAR PYROLYSE CATALYTIQUE DE BIOMASSE

(30) Priority: 21.02.2017 CN 201710092409
(43) Date of publication of application: 01.01.2020
(73) Proprietor: North China Electric Power University, Beijing 102206 (CN)
(72) Inventor: LU, Qiang, Beijing 102206 (CN); YE, Xiaoning, Beijing 102206 (CN); WANG, Xin, Beijing 102206 (CN); GUO, Haoqiang, Beijing 102206 (CN); PENG, Li, Beijing 102206 (CN); DONG, Changqing, Beijing 102206 (CN); YANG, Yongping, Beijing 102206 (CN)
(74) Representative: Huang, Liwei
(86) International application number: PCT/CN2017/075981
(87) International publication number: WO 2018/152870

(56) References cited:
- EP-A2- 0 416 577
- WO-A1-2008/098036
- WO-A1-2016/039996
- WO-A1-2016/170329
- CN-A- 102 532 206
- CN-A- 102 816 187
- CN-B- 102 816 187
- US-B1- 9 376 451
- GIRGIS B S ET AL: "Activated carbon from sugar cane bagasse by carbonization in the presence of inorganic acids", JOURNAL OF CHEMICAL TECHNOLOGY AND BIOTECHNOLOGY, WILEY, vol. 61, no. 1, 1 September 1994 (1994-09-01), pages 87-92, XP008104369, ISSN: 0268-2575, DOI: 10.1002/JCTB.280610113 [retrieved on 2004-02-09]
- G DOBELE ET AL: "Pre-treatment of biomass with phosphoric acid prior to fast pyrolysis", JOURNAL OF ANALYTICAL AND APPLIED PYROLYSIS, vol. 68-69, 28 March 2003 (2003-03-28), pages 197-211, XP055651633, NL ISSN: 0165-2370, DOI: 10.1016/S0165-2370(03)00063-9
- XIAN-WEI SUI ET AL: "Preparation of levoglucosenone through sulfuric acid promoted pyrolysis of bagasse at low temperature", BIORESOURCE TECHNOLOGY, ELSEVIER, AMSTERDAM, NL, vol. 103, no. 1, 3 October 2011 (2011-10-03), pages 466-469, XP028120898, ISSN: 0960-8524, DOI: 10.1016/J.BIORTECH.2011.10.010 [retrieved on 2011-10-12]
- ZHI-BO ZHANG ET AL: "Selective Production of Levoglucosenone from Catalytic Fast Pyrolysis of Biomass Mechanically Mixed with Solid Phosphoric Acid Catalysts", BIOENERGY RESEARCH, vol. 8, no. 3, 5 February 2015 (2015-02-05), pages 1263-1274, XP055229070, Boston ISSN: 1939-1234, DOI: 10.1007/s12155-015-9581-6

## Description

### Field of the invention

The invention belongs to the field of biomass energy utilization, and particularly relates to a method for preparing levoglucosenone by catalytic pyrolysis of biomass.

### Background of the invention

Levoglucosenone (LGO, 1,6-anhydro-3,4-dideoxy-β-D-pyranose-2-one) is an important anhydrosugar derivative generated through dehydration reactions of cellulose and partial hemicellulose during biomass pyrolysis process. It is highly reactive, and an important raw material for organic synthesis, which has high value of application.

It has been more than 40 years since levoglucosenone was first discovered in 1973. However, due to its low yield in conventional pyrolysis of biomass and the difficulty in its purification, the market price of levoglucosenone is expensive, restraining its application in organic synthesis. Previous studies have verified that introducing acid catalysts into the pyrolysis of cellulose or biomass significantly promoted the formation of levoglucosenone. Currently, the effective catalysts that have been reported include liquid acids (phosphoric acid (J. Anal. Appl. Pyrolysis (2003) 68-69: 197-211), sulfuric acid (Bioresource Technology (2012) 103(1): 466-469)), ionic liquids, solid acids (solid phosphoric acid (Bioenerg. Res. (2015) 8(3): 1263-1274), solid super acid) and so on. Nevertheless, among the mentioned catalysts, liquid acids need to be impregnated into cellulose or biomass raw materials through cumbersome pretreatment steps. Ionic liquids are expensive but in poor thermal stability. Moreover, these acid catalysts are in high acidity, due to the presence of water vapor, a considerable amount of acid sites will release into the pyrolytic liquid products, which will catalyze the secondary reactions of levoglucosenone, resulting in the decrease of levoglucosenone. In addition, after catalytic pyrolysis reactions, the catalyst and solid char are mixed together, which limits the recovery of catalyst as well as the utilization of solid char. Besides, the large use of these strong acid catalysts will lead to environmental issues. Therefore, it is necessary to develop a novel green method for environmentally friendly, low-cost and efficient preparation of levoglucosenone.

CN 102816187B discloses a method for preparing levoglucosenone which uses char sulfonic acid (also named as "carbon-based sulfonic acid") as the catalyst, wherein the char sulfonic acid is produced by immersing biomass char (which was produced by pyrolysis from biomass raw materials) into concentrated sulfuric acid at a temperature of above 100°C for sulfonation.

GIRGIS B S ET AL: "Activated carbon from sugar cane bagasse by carbonization in the presence of inorganic acids", JOURNAL OF CHEMICAL TECHNOLOGY AND BIOTECHNOLOGY, vol. 61, no. 1, 1994, pages 87-92, discloses a method of preparing activated carbon by using phosphoric acid as an activating agent. However, GIRGIS B S ET AL does not mention the use of this biomass-based activated carbon as a catalyst in catalytic pyrolysis of biomass.

### Detailed description of the invention

The objective of this invention is to overcome the deficiencies of current technologies, and provide a method for preparing levoglucosenone from catalytic pyrolysis of biomass.

The present invention is a method for preparing levoglucosenone from catalytic pyrolysis of biomass as defined in claim 1, as well as some advantageous embodiments as defined in the dependent claims.

This method utilizes biomass-based activated carbon which has been activated by phosphoric acid as the catalyst and lignocellulosic biomass as the raw material. The catalyst and raw material are mechanically mixed in mass ratios of (5:1)-(1:10), and then subjected to fast pyrolysis under inert atmosphere in the absence of oxygen at 250-470°C. The generated pyrolytic vapors are collected. The pyrolysis reaction lasts no more than 50s. The pyrolytic vapors are condensed to obtain a liquid product rich in levoglucosenone.

The biomass-based activated carbon which has been activated by phosphoric acid is prepared as the following steps. Biomass raw material is ground to obtain particles less than 1mm. The particles are impregnated in 10-80wt% phosphoric acid solvent for 0.5-2h. After drying at 110°C for 12-24h to remove the free water, a precursor on which the phosphoric acid loading ranging from 5 to 70wt% is obtained. The precursor is activated under inert oxygen-free atmosphere. After cooling down, the solid is washed by deionized water to neutral. After drying, the biomass-based activated carbon which has been activated by phosphoric acid is obtained.

Preferably, the activation condition includes a heating rate of 5-20°C/min, an activation temperature of 350-750°C, and an activation time of 0.5-5h.

Preferably, the temperature of deionized water is 40-80°C.

Preferably, the biomass raw material includes lignocellulosic biomass and carbohydrates.

Said lignocellulosic biomass includes wood, crop straw, bamboo or herbaceous biomass.

Said inert oxygen-free atmosphere is using nitrogen (N₂), or argon (Ar), or other inert gases as the carrier gas.

The heating rate of said pyrolysis reaction is higher than 100°C/s.

The beneficial effects of this invention include:
This invention utilizes biomass-based activated carbon which has been activated by phosphoric acid as the catalyst, which is mechanically mixed with biomass raw materials for catalytic pyrolysis under relatively low temperatures, to obtain a liquid product rich in levoglucosenone. Activated carbon is a common and widely used carbon-based material. Its preparation methods mainly include physical activation method, chemical activation method, as well as combined physical and chemical activation method. Phosphoric acid activation is a common chemical activation method for preparing activated carbon. Therefore, the catalyst used in this invention is commonly available and low in cost. In addition, the activated carbon catalyst used in this invention is prepared from the biomass raw material, bearing the characteristics of green and environmentally friendly. Overall, compared with various strong acid catalysts, the activated carbon has obvious advantages.

The greatest beneficial effect of this invention is to avoid using strong acid catalysts for levoglucosenone preparation. The strong acid catalysts (sulfuric acid, ionic liquid, and solid super acid) reported in previous studies on levoglucosenone preparation will bring environmental issues. Moreover, during catalytic pyrolysis of biomass process, the existence of a large amount of water vapor results in the loss of catalyst or its acid sites. These effluent acid species are eventually condensed into the liquid product along with the pyrolytic vapors. Due to their strong catalytic effects, the acid species will catalyze the secondary reactions of lcvoglucoscnonc, leading to the decrease in levoglucosenone yield, as well as the great difficulties in storage of the liquid product. Compared with the strong acid catalysts (achieving catalytic pyrolysis for levoglucosenone preparation through massive acid sites), the biomass-based activated carbon which has been activated by phosphoric acid is nearly neutral (the washing step in preparation removes the phosphoric acid and its derivatives from the activated carbon), which relies on only a small amount of acid sites (functional groups such as C-O-P and C-P) to achieve efficient and selective preparation of levoglucosenone. The activated carbon which has been activated by phosphoric acid has significant differences with the aforementioned strong acid catalysts in chemical composition and catalytic mechanism. The activated carbon which has been activated by phosphoric acid as a neutral catalyst, relying on only a small amount of active sites such as C-O-P and C-P, can achieve high efficiency and selectivity for levoglucosenone preparation, which has obvious technical advantages. More importantly, the yield and selectivity of levoglucosenone from catalytic pyrolysis using biomass-based activated carbon which has been activated by phosphoric acid are higher than the reported strong acid catalysts.

In addition, using activated carbon as the catalyst is more convenient than other strong acid catalysts, which is introduced into the pyrolysis system by only mechanically mixing with biomass raw materials, thereby avoiding cumbersome and energy-consuming steps such as impregnation and drying when using a liquid acid catalyst. After the catalytic pyrolysis reaction, the activated carbon catalyst is mixed with the biomass pyrolytic char. Since the activated carbon itself is a carbon material derived from biomass, similar in nature with biomass pyrolytic char, it will not affect the subsequent use of the biomass pyrolytic char. Moreover, the solid mixture can also be used as a precursor of activated carbon preparation, to be further activated for catalytic pyrolysis of biomass to prepare levoglucosenone.

### Examples

The invention provides a method for preparing levoglucosenone by catalytic pyrolysis of biomass as defined by the appended claims. The invention is further described below in conjunction with specific embodiments.

The percentages in the following examples are all by mass unless otherwise stated.

### Example 1

100g of dried poplar (particle size within 0.1-0.3mm) was used as the raw material, immersed in 70% phosphoric acid solution for 1h, and dried at 110°C for 18h to remove the free water. The loading of phosphoric acid was 45.8%. Then under inert atmosphere the impregnated solid was heated from room temperature to 500°C in 10°C/min, and maintained at 500°C for 2h to activate. After cooled down, the activated solid was washed using 60°C deionized water to neutral, dried and then 27g biomass-based activated carbon which has been activated by phosphoric acid was obtained.

10g of the above biomass-based activated carbon which has been activated by phosphoric acid was ground to average particle diameter about 0.2mm, then was mechanically mixed with the poplar (particle size within 0.1-0.3mm). The mass ratio of poplar to catalyst was 5:1. The mixture was pyrolyzed at 330°C, a heating rate of more than 500°C/s, and a N₂ atmosphere for 15s, obtaining a liquid product in a yield of 43.6%. The content of levoglucosenone was analyzed by gas chromatography, and the yield of levoglucosenone was calculated to be 8.5%.

### Example 2

5g of the biomass-based activated carbon which has been activated by phosphoric acid in Example 1 was ground to average particle diameter about 0.2mm, then was mechanically mixed with the pine (particle size within 0.1-0.3mm). The mass ratio of pine to catalyst was 4:1. The mixture was pyrolyzed at 330°C, a heating rate of more than 500°C/s, and a N₂ atmosphere for 20s, obtaining a liquid product in a yield of 41.6%. The content of levoglucosenone was analyzed by gas chromatography, and the yield of levoglucosenone was calculated to be 9.9%.

### Example 3

7g of the biomass-based activated carbon which has been activated by phosphoric acid in Example 1 was ground to average particle diameter about 0.2mm, then was mechanically mixed with the corn stalk (particle size within 0.1-0.3mm). The mass ratio of corn stalk to catalyst was 3:1. The mixture was pyrolyzed at 310°C, a heating rate of more than 500°C/s, and an Ar atmosphere for 20s, obtaining a liquid product in a yield of 41.9%. The content of levoglucosenone was analyzed by gas chromatography, and the yield of levoglucosenone was calculated to be 8.2%.

### Example 4

50g of dried bagasse (particle size within 0.1-0.3mm) was used as the raw material, immersed in 75% phosphoric acid solution for 2h, and dried at 110°C for 24h to remove the free water. The loading of phosphoric acid was 52.6%. Then under inert atmosphere the impregnated solid was heated from room temperature to 450°C in 8°C/min, and maintained at 450°C for 1.5h to activate. After cooled down, the activated solid was washed using 65°C deionized water to neutral, dried and then 14g biomass-based activated carbon which has been activated by phosphoric acid was obtained.

8g of the above biomass-based activated carbon which has been activated by phosphoric acid was ground to average particle diameter about 0.2mm, then was mechanically mixed with the bagasse (particle size within 0.1-0.3mm). The mass ratio of bagasse to catalyst was 2:1. The mixture was pyrolyzed at 350°C, a heating rate of more than 1000°C/s, and a N₂ atmosphere for 15s, obtaining a liquid product in a yield of 46.7%. The content of levoglucosenone was analyzed by gas chromatography, and the yield of levoglucosenone was calculated to be 7.4%.

### Example 5

3g of the biomass-based activated carbon which has been activated by phosphoric acid in Example 4 was ground to average particle diameter about 0.2mm, then was mechanically mixed with the pine (particle size within 0.1-0.3mm). The mass ratio of pine to catalyst was 3:1. The mixture was pyrolyzed at 310°C, a heating rate of more than 800°C/s, and a N₂ atmosphere for 15s, obtaining a liquid product in a yield of 42.1%. The content of levoglucosenone was analyzed by gas chromatography, and the yield of levoglucosenone was calculated to be 10.4%.

### Example 6

4g of the biomass-based activated carbon which has been activated by phosphoric acid in Example 4 was ground to average particle diameter about 0.2mm, then was mechanically mixed with the bamboo leaves (particle size within 0.1-0.3mm). The mass ratio of bamboo leaves to catalyst was 5:1. The mixture was pyrolyzed at 290°C, a heating rate of more than 600°C/s, and an Ar atmosphere for 20s, obtaining a liquid product in a yield of 38.5%. The content of levoglucosenone was analyzed by gas chromatography, and the yield of levoglucosenone was calculated to be 8.0%.

### Example 7

200g of dried pine (particle size within 0.1-0.3mm) was used as the raw material, immersed in 60% phosphoric acid solution for 1h, and dried at 110°C for 12h to remove the free water. The loading of phosphoric acid was 40.2%. Then under inert atmosphere the impregnated solid was heated from room temperature to 550°C in 15°C/min, and maintained at 550°C for 2h to activate. After cooled down, the activated solid was washed using 55°C deionized water to neutral, dried and then 55g biomass-based activated carbon which has been activated by phosphoric acid was obtained.

20g of the above biomass-based activated carbon which has been activated by phosphoric acid was ground to average particle diameter about 0.2mm, then was mechanically mixed with the poplar (particle size within 0.1-0.3mm). The mass ratio of poplar to catalyst was 2:1. The mixture was pyrolyzed at 370°C, a heating rate of more than 500°C/s, and a N₂ atmosphere for 10s, obtaining a liquid product in a yield of 52.2%. The content of levoglucosenone was analyzed by gas chromatography, and the yield of levoglucosenone was calculated to be 7.1%.

### Example 8

5g of the biomass-based activated carbon which has been activated by phosphoric acid in Example 7 was ground to average particle diameter about 0.2mm, then was mechanically mixed with the pine (particle size within 0.1-0.3mm). The mass ratio of pine to catalyst was 5:1. The mixture was pyrolyzed at 300°C, a heating rate of more than 700°C/s, and a N₂ atmosphere for 20s, obtaining a liquid product in a yield of 39.8%. The content of levoglucosenone was analyzed by gas chromatography, and the yield of levoglucosenone was calculated to be 9.5%.

### Example 9

10g of the biomass-based activated carbon which has been activated by phosphoric acid in Example 7 was ground to average particle diameter about 0.2mm, then was mechanically mixed with the rice straw (particle size within 0.1-0.3mm). The mass ratio of rice straw to catalyst was 3:1. The mixture was pyrolyzed at 330°C, a heating rate of more than 1000°C/s, and an Ar atmosphere for 15s, obtaining a liquid product in a yield of 44.3%. The content of levoglucosenone was analyzed by gas chromatography, and the yield of levoglucosenone was calculated to be 8.1%.

The above examples are preferred embodiments of this invention, and are not intended to limit this invention.

## Claims

1. A method for preparing levoglucosenone from catalytic pyrolysis of biomass, wherein a neutral catalyst of biomass-based activated carbon, which has been activated by phosphoric acid and then washed to neutral, is used as the catalyst, and lignocellulosic biomass is raw material, the method comprising:
mechanically mixing the catalyst and raw material in mass ratio of (5:1)-(1:10),
fast pyrolysis reaction under inert oxygen-free atmosphere at 250-470°C, wherein the pyrolysis reaction lasts no more than 50s,
collecting pyrolytic vapors and condensing to produce a liquid product rich in levoglucosenone;
wherein said catalyst is prepared by the following steps:
grinding biomass raw material to obtain particles less than 1mm,
impregnating said particles in 10-80wt% phosphoric acid solvent for 0.5-2h at room temperature,
drying at 110°C for 12-24h to remove the free water, obtaining the precursor, wherein the phosphoric acid loading is controlled to be from 5 to 70wt%,
activating said precursor under inert atmosphere,
after cooling, washing to neutral using deionized water,
drying to obtain the activated carbon catalyst.

2. The method according to claim 1, wherein in the activating process, the heating rate is 5-20°C/min, the activation temperature is 350-750°C, and the activation time is 0.5-5h.

3. The method according to claim 1, wherein the temperature of said deionized water for washing is 40-80°C.

4. The method according to claim 1, wherein said biomass raw material is a mixture of lignocellulosic biomass and carbohydrates.

5. The method according to claim 1, wherein said lignocellulosic biomass includes wood, crop straw, bamboo or herbaceous biomass.

6. The method according to claim 1, wherein said inert oxygen-free atmosphere is using nitrogen (N₂) or argon (Ar) as the carrier gas.

7. The method according to claim 1, wherein the heating rate of said pyrolysis reaction is higher than 100°C/s.

## Patentansprüche

1. Verfahren zur Herstellung von Levoglucosenon aus der katalytischen Pyrolyse von Biomasse, wobei ein neutraler Katalysator aus Aktivkohle auf der Basis von Biomasse, der durch Phosphorsäure aktiviert und dann zu neutral gewaschen wurde, als Katalysator eingesetzt wird, und wobei Lignozellulose-haltige Biomasse Rohmaterial ist, wobei das Verfahren umfasst:
mechanisches Mischen des Katalysators und des Rohmaterials in einem Massenverhältnis von (5:1)-(1:10),
schnelle Pyrolyse-Reaktion in sauerstofffreier Schutzgasatmosphäre bei 250-470°C, wobei die Pyrolyse-Reaktion nicht länger als 50 s dauert,
Auffangen pyrolytischer Dämpfe und Kondensation zur Herstellung eines flüssigen Produktes, das reich an Levoglucosenon ist,
wobei der Katalysator durch die folgenden Schritte hergestellt wird:
Mahlen des Biomasse-Rohmaterials zum Erhalt von Partikeln, die kleiner als 1 mm sind,
Imprägnieren der Partikel in 10-80 Gew.-% Lösemittel aus Phosphorsäure über 0,5 - 2 h bei Raumtemperatur,
Trocknen bei 110°C über 12-24 h zum Entfernen des freien Wassers, Erhalt des Vorläufer-Produktes, wobei der Anteil der Phosphorsäure derart gesteuert wird, dass er bei 5 bis 70 Gew.-% liegt,
Aktivierung des Vorläufer-Produktes in Schutzgasatmosphäre,
nach dem Abkühlen, Waschen zu neutralem Zustand unter Verwendung von entionisiertem Wasser,
Trocknen zum Erhalt des Aktivkohlekatalysators.

2. Verfahren gemäß Anspruch 1, wobei bei dem Aktivierungsprozess die Aufheizgeschwindigkeit 5-20°C/min beträgt, die Aktivierungstemperatur bei 350-750°C liegt und die Aktivierungszeit 0,5-5 h beträgt.

3. Verfahren gemäß Anspruch 1, wobei die Temperatur des entionisierten Wassers zum Waschen 40-80°C beträgt.

4. Verfahren gemäß Anspruch 1, wobei das Biomasse-Rohmaterial ein Gemisch aus Lignozellulose-haltiger Biomasse und Kohlehydraten ist.

5. Verfahren gemäß Anspruch 1, wobei die Lignozellulose-haltige Biomasse Holz, Getreidestroh, Bambus oder kräuterartige Biomasse umfasst.

6. Verfahren gemäß Anspruch 1, wobei die sauerstofffreie Schutzgasatmosphäre Stickstoff (N₂) oder Argon (Ar) als Trägergas verwendet.

7. Verfahren gemäß Anspruch 1, wobei die Aufheizgeschwindigkeit der Pyrolysereaktion höher als 100°C/s ist.

## Revendications

1. Procédé de préparation de la lévoglucosénone à partir d'une pyrolyse catalytique de biomasse, dans lequel un catalyseur neutre en charbon activé à base de biomasse, qui a été activé par l'acide phosphorique et ensuite lavé pour obtenir l'état neutre, est utilisé comme catalyseur, et de la biomasse contenant de la lignocellulose est la matière première, le procédé comprenant les étapes de :
mélanger mécaniquement le catalyseur et la matière première dans un rapport de masse de (5:1)-(1:10),
une réaction de pyrolyse rapide dans une atmosphère inerte sans oxygène à 250-470°C, dans lequel la réaction de pyrolyse ne dure pas plus de 50 secondes,
collecter des vapeurs pyrolytiques et les condenser pour produire un produit liquide, qui est riche en lévoglucosénone ;
dans lequel ledit catalyseur est préparé dans les étapes suivantes de :
grincer la matière première de biomasse pour obtenir des particules inférieures à 1 mm,
imprégner lesdites particules dans 10-80 % en poids d'un solvant d'acide phosphorique pendant 0,5-2 h, à température ambiante,
sécher à 110°C pendant 12-24 h pour enlever l'eau libre, obtenir le précurseur, dans lequel on fixe la proportion de l'acide phosphorique dans une gamme comprise entre 5 et 70 % en poids,
activer ledit précurseur dans une atmosphère inerte,
après refroidissement, laver pour obtenir l'état neutre en utilisant de l'eau désionisée,
sécher pour obtenir la catalyseur en charbon activé.

2. Procédé selon la revendication 1, dans lequel la vitesse de chauffage est 5-20°C/min, la température d'activation est 350-750°C et la durée d'activation est 0,5-5 h pendant la procédure d'activation.

3. Procédé selon la revendication 1, dans lequel la température de l'eau désionisée pour le lavage est comprise entre 40 et 80°C.

4. Procédé selon la revendication 1, dans lequel ladite matière première de biomasse est un mélange d'une biomasse contenant de la lignocellulose et des carbohydrates.

5. Procédé selon la revendication 1, dans lequel ladite biomasse contenant de la lignocellulose comprend du bois, de la paille de culture, du bambou ou de la biomasse herbacée.

6. Procédé selon la revendication 1, dans lequel l'atmosphère inerte sans oxygène utilise de l'azote (N₂) ou de l'argon (Ar) comme gaz porteur.

7. Procédé selon la revendication 1, dans lequel la vitesse de chauffage de ladite réaction de pyrolyse est supérieure à 100°C/s.
